# FASCICULE DE BREVET EUROPEEN

(11) **EP 0 475 825 B1**
(45) Date de publication et mention de la délivrance du brevet: **23.07.1997**
(21) Numéro de dépôt: 91402380.9
(22) Date de dépôt: 05.09.1991
(51) Int. Cl.: A61M 1/14

(54) **Dispositif pour la préparation extemporanée et en continu de dialysat**
Vorrichtung zur extemporierten und kontinuierlichen Präparation eines Dialysats
Device for the extemporaneous and continuous preparation of dialysates

(30) Priorité: 13.09.1990 FR 9011578
(43) Date de publication de la demande: 18.03.1992
(73) Titulaire: SOLUDIA, S.A., F-31450 Montgiscard (FR); RENACARE LIMITED, Sutton-in-Ashfield, Nottinghamshire NG17 2HU (GB)
(72) Inventeur: Lascombes, Jean-Jacques, F-31000 Toulouse (FR); Pujo, Jean-Michel, F-31140 Caraman (FR)
(74) Mandataire: Morelle, Guy Georges Alain

(56) Documents cités:
- EP-A- 0 469 487
- FR-A- 1 410 896

## Description

La présente invention a pour objet un dispositif pour la préparation extemporanée et en continu d'une solution de dialyse par dissolution d'un composé solide, sous forme granuleuse ou pulvérulente, dans de l'eau purifiée.

De tels dispositifs sont associés à des générateurs d'hémodialyse qui commandent leur fonctionnement. On connait depuis quelque temps déjà des matériels de ce genre. Ainsi, la demande de Brevet français No. 2.604.922 décrit un dispositif qui est constitué schématiquement d'une cuve de mélange fixe munie d'une conduite d'alimentation en eau et d'une conduite de sortie du liquide de dialyse reliées à un générateur. Dans ladite cuve est introduite une quantité déterminée de composé sous forme solide délivrée à partir d'un réservoir de stockage au moyen d'un système doseur, le mélange étant assuré par un dispositif d'agitation.

Outre la complexité de sa structure, un tel dispositif nécessite de trop nombreuses manipulations du composé, celui-ci étant d'abord extrait de son emballage pour être stocké en vrac dans une trémie d'alimentation avant d'être déversé dans la cuve de mélange.

Le dispositif, objet de l'invention, ne présente pas de tels inconvénients. Il autorise en effet un conditionnement préalable du composé solide dans un volume étanche, selon des doses pré-déterminées nécessaires pour effectuer une ou plusieurs opérations de dialyse, ledit volume étant ensuite lui-même utilisé pour la préparation de la solution.

On connaît par ailleurs par l'EP-A-0.469.487, un appareil pour dissoudre un agent d'une solution de dialyse, par exemple du bicarbonate de sodium, comprenant un récipient contenant ledit agent sous forme solide, des moyens d'alimentation en fluide, ledit fluide étant destiné à dissoudre ledit agent, et une cuve de dissolution munie d'un agitateur ou d'un circuit de recirculation pour mélanger et dissoudre ledit agent avec ledit fluide et pour conserver la solution obtenue.

Avec un tel appareil, la dissolution de l'agent n'est pas opérée directement dans le récipient, qui sert en fait à opérer des prélévements de quantités déterminées d'agent, mais dans la cuve qui peut être reliée à une unité pour la préparation d'une solution de dialyse. Un tel appareil ne permet pas ainsi de relier directement le récipient contenant l'agent sous forme solide à un générateur de dialyse car ce récipient n'est pas conçu pour permettre d'obtenir une dissolution totale de l'agent dans le récipient lui-même.

Le dispositif, objet de l'invention, permet d'atteindre cet objectif. Plus précisément, l'invention a pour objet un dispositif pour la préparation extemporanée et en continu de dialysat, obtenu à partir d'une solution concentrée et/ou saturée préparée par dissolution dans un conteneur d'un composé solide avec de l'eau traitée, ledit conteneur étant muni d'un orifice d'entrée pour l'eau traitée, relié de manière amovible à la sortie d'eau d'un générateur de dialyse, et d'un orifice de sortie pour la solution de dialyse, relié de manière amovible à l'entrée de dialysat dudit générateur, ledit dispositif étant remarquable en ce que les susdits orifices d'entrée pour l'eau traitée et de sortie pour la solution de dialyse sont situés sur un même niveau du susdit conteneur, ce dernier comportant des moyens de diffusion de l'eau dans le volume de composé solide et sa paroi interne présentant au moins une portion inclinée pour éviter l'accumulation du composé solide.

On comprend sans difficulté l'intérêt procuré par un tel dispositif. Pour réaliser une opération de dialyse, il suffit en effet de relier de manière étanche un conteneur du type ci-dessus aux tubulures de sortie d'eau et d'entrée de dialysat équipant un générateur, ce conteneur renfermant la dose de produits solides nécessaire pour une telle opération. Après dissolution de la dose de composé dans le conteneur et aspiration de la solution obtenue, on désaccouple le générateur et le conteneur utilisé pour éventuellement mettre en place un nouveau conteneur. Il résulte de l'emploi d'un tel dispositif un gain évident de temps et une grande économie de moyens.

D'autres caractéristiques et avantages de l'invention ressortiront à la lecture de la description qui suit de deux exemples de réalisation d'un dispositif conforme aux enseignements ci-dessus donnés, ces exemples étant donnés à titre simplement d'illustration et sans qu'aucune interprétation restrictive de la protection recherchée puisse en être tirée.

Cette description est accompagnée de dessins où :
La Figure 1 représente, selon une vue en perspective partiellement écorchée, un premier mode de réalisation d'un conteneur destiné à être raccordé à un générateur de dialyse qu'il n'a pas été jugé utile de représenter dans la mesure où un tel appareil est bien connu des spécialistes ;
La Figure 2 représente, selon une vue en coupe schématique, un second mode de réalisation d'un conteneur destiné également à être raccordé à un générateur de dialyse.

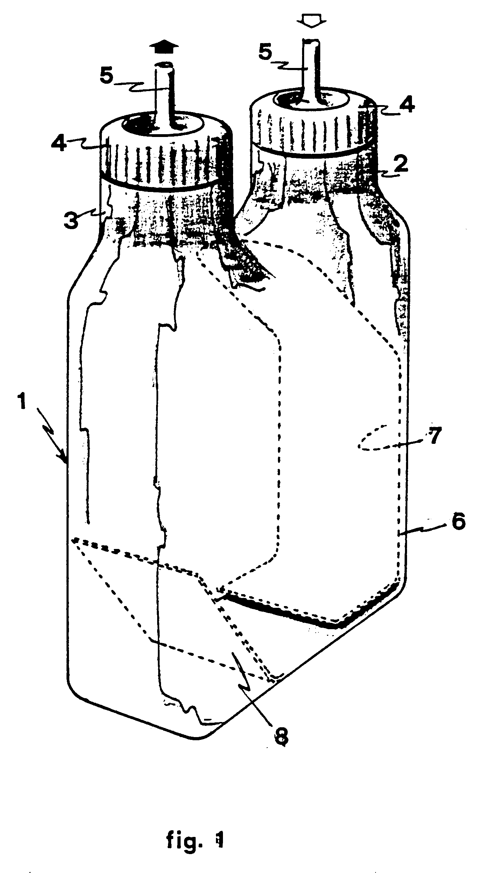

Le conteneur 1, illustré à la Figure 1, offre l'aspect d'une bouteille de forme sensiblement parallélépipédique, qui est munie de deux goulots parallèles identiques. Le conteneur comporte en effet deux ouvertures situées sur un même niveau en partie haute, l'une 2 servant d'entrée pour l'eau purifiée, l'autre 3 étant destinée à la sortie du liquide de dialyse obtenu après dissolution du composé solide renfermé dans le conteneur. Dans le mode réalisation donné en exemple, le conteneur illustré est plus particulièrement destiné au conditionnement et à la dissolution d'un composé sous forme de poudre.

Ces ouvertures 2 et 3 sont obturées par des bouchons étanches 4, équipés d'un filtre d'une porosité comprise entre 1 et 100 µ, chaque bouchon étant muni sur sa partie sommitale d'un conduit souple 5 sur lequel pourra être monté un système régulateur de débit. Ces conduits souples 5 servent ainsi de moyens de raccordement au générateur de dialyse.

Afin de faciliter la diffusion de l'eau dans le volume de composé solide, le conteneur 1 comprend une conduite 6 destinée à canaliser le flux d'eau en partie basse dudit conteneur. Dans le mode de réalisation illustré, il s'agit d'une conduite disposée à l'intérieur du conteneur et qui est formée par une portion de la paroi du conteneur reliant les parties haute et basse de ce dernier et par une cloison interne 7, représentée en traits mixtes, qui s'étend à partir des bords de l'orifice d'entrée d'eau 2 de manière sensiblement parallèle à ladite portion de paroi.

Comme on peut en outre l'observer, cette conduite 6 débouche dans le fond du conteneur, sensiblement au milieu de la paroi de fond, en regard d'une portion inclinée 8 de ladite paroi. Cette portion inclinée constitue également un moyen permettant d'obtenir une meilleure diffusion de l'eau dans le volume de poudre mais a surtout pour rôle d'éviter l'accumulation de poudre dans l'angle correspondant. A cet effet, l'angle formé entre le fond du conteneur 1 et la portion de paroi inclinée 8 faisant face à la sortie de la conduite interne 6 est de préférence compris entre 150 et 130 °.

Ainsi, l'eau entrant dans le conteneur par l'orifice 2 est amenée par la conduite 6 au bas du volume de poudre et se diffuse au travers de celui-ci pour se charger en composés. Une solution concentrée et saturée se forme alors dans la partie supérieure du conteneur et est ensuite extraite par aspiration au travers de l'orifice de sortie 3.

Le conteneur illustré possède une capacité de 2 litres. Mais il est tout à fait possible de réaliser des conteneurs présentant les mêmes caractéristiques structurelles, offrant une capacité de 1 à 10 litres et contenant 0,5 à 6 Kg de composé solide sous forme de poudre.

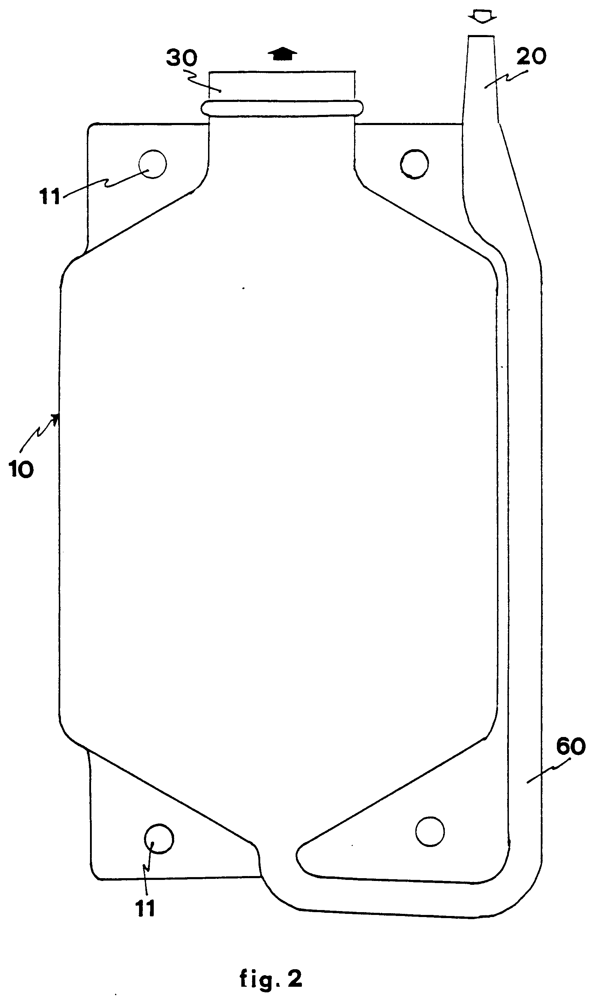

Le conteneur 10, illustré selon une vue en coupe à la Figure 2, présente également l'aspect d'une bouteille dont les parois internes forment entre elles des angles d'environ 130°, ceci afin d'éviter la formation de petits tas de poudre dans les angles inférieurs du conteneur.

Il est muni d'un orifice d'entrée 20 pour l'eau traitée et d'un orifice de sortie 30 pour la solution de dialyse. Comme on peut le voir, l'orifice d'entrée 20 est associé à une conduite externe 60 qui débouche sensiblement au milieu de la paroi de fond du conteneur entre deux portions inclinées.

Un tel conteneur peut être utilisé comme celui de la Figure 1 dans la position telle que représentée, c'est-à-dire que les orifices d'entrée 20 et de sortie 30 sont disposés en partie haute du conteneur. Mais il peut être utilisé également, selon le type de générateur de dialyse auquel il est raccordé, dans une position inversée de 180°, les orifices d'entrée 20 et de sortie 30 étant disposés alors en partie basse et l'eau traitée étant ainsi amenée par la conduite 60 en haut du volume de poudre. Cette dernière position présente notamment l'avantage d'éviter la formation de bulles d'air au niveau de la sortie de la solution de dialyse.

Pour permettre l'emploi du conteneur indifféremment dans l'une ou l'autre position, ce dernier est muni d'oeillets de suspension 11 situés à ses quatre angles.

Les conteneurs illustrés aux Figures 1 et 2 seront de préférence obtenus par moulage d'une matière plastique dont les qualités physico-chimiques seront appropriées à l'usage particulier desdits conteneurs.

## Revendications

1. Dispositif pour la préparation extemporanée et en continu de dialysat, obtenu à partir d'une solution concentrée et/ou saturée préparée par dissolution dans un conteneur (1, 10) d'un composé solide avec de l'eau traitée, ledit conteneur étant muni d'un orifice d'entrée (2, 20) pour l'eau traitée, relié de manière amovible à la sortie d'eau d'un générateur de dialyse, et d'un orifice de sortie (3, 30) pour la solution de dialyse, relié de manière amovible à l'entrée de dialysat dudit générateur, les susdits orifices d'entrée (2, 20) pour l'eau traitée et de sortie (3, 30) pour la solution de dialyse étant situés sur un même niveau de susdit conteneur (1, 10), ***caractérisé en ce que*** ce dernier comportant des moyens de diffusion de l'eau dans le volume de composé solide et sa paroi interne présentant au moins une portion inclinée (8) pour éviter l'accumulation du composé solide.

2. Dispositif selon la Revendication 1, ***caractérisé en ce que*** les susdits moyens de diffusion de l'eau dans le volume de composé solide sont constitués par une conduite (6, 60).

3. Dispositif selon la Revendication 2, ***caractérisé en ce que*** la susdite conduite (6) est disposée à l'intérieur dudit conteneur (1).

4. Dispositif selon la Revendication 2, ***caractérisé en ce que*** la susdite conduite (60) est disposée à l'extérieur dudit conteneur (10).

5. Dispositif selon la Revendication 1, ***caractérisé en ce que*** ladite portion inclinée (8) forme un angle compris entre 150 et 130° avec la paroi interne du conteneur.

6. Dispositif selon la Revendication 2, ***caractérisé en ce que*** ladite conduite (6) est formée par une portion de la paroi du conteneur reliant les parties haute et basse de ce dernier et par une cloison interne (7) de celui-ci s'étendant à partir des bords du susdit orifice d'entrée d'eau (2) de manière sensiblement parallèle à ladite portion de paroi.

7. Dispositif selon la Revendication 1, ***caractérisé en ce que*** les orifices d'entrée (2, 20) pour l'eau traitée et de sortie (3, 30) pour la solution de dialyse sont munis d'un filtre d'une porosité comprise entre 1 et 100 µ.

8. Dispositif selon l'une quelconque des Revendications précédentes, ***caractérisé en ce que*** ledit conteneur a une capacité de 1 à 10 litres et contient 0,5 à 6 Kg de composé solide sous forme de poudre.

## Claims

1. A device for the extemporaneous, continuous preparation of dialysate, obtained from a concentrated and/or saturated solution prepared by the dissolution of a solid compound in processed water in a container (1, 10), the said container being provided with an inlet orifice (2, 20) for the processed water connected in a removable manner to the water outlet of a dialysis generator, and with an outlet orifice (3, 30) for the dialysis solution connected in a removable manner to the dialysate inlet of the said generator, the said inlet orifice (2, 20) for the processed water and the said outlet orifice (3, 30) for the dialysis solution being situated at the same level of the said container (1, 10), characterised in that the container comprises means for distributing water within the volume of the solid compound and its internal wall has at least one inclined portion (8) to prevent the accumulation of the solid compound.

2. A device according to claim 1, characterised in that the said means for distributing water within the volume of the solid compound are formed by a duct (6, 60).

3. A device according to claim 2, characterised in that the said duct (6) is disposed inside the said container (1).

4. A device according to claim 2, characterised in that the said duct (60) is disposed outside the said container (10).

5. A device according to claim 1, characterised in that the said inclined portion (8) forms an angle between 150 and 130° with the internal wall of the container.

6. A device according to claim 2, characterised in that the said duct (6) is formed by a portion of the wall of the container which connects the top and bottom parts of the latter and by an internal partition (7) of the container extending from the edges of the said water inlet orifice (2) substantially parallel to the said portion of wall.

7. A device according to claim 1, characterised in that the inlet orifice (2, 20) for the processed water and the outlet orifice (3, 30) for the dialysis solution are provided with a filter with a porosity between 1 and 100 µ.

8. A device according to any one of the preceding claims, characterised in that the said container has a capacity from 1 to 10 litres and contains 0.5 to 6 kg of solid compound in powder form.

## Patentansprüche

1. Vorrichtung zur extemporierten und kontinuierlichen Bereitung von Dialysat, das ausgehend von einer konzentrierten und/ oder gesättigten Lösung erhalten wird, welche durch Auflösen in einem Behälter (1, 10) von einer festen Verbindung mit dem behandelten Wasser hergestellt wird, wobei der Behälter mit einer Einlaßöffnung (2, 20) für das behandelte Wasser versehen ist, welche in lösbarer Weise mit dem Wasserausgang eines Dialysegenerators verbunden ist, sowie mit einer Auslaßöffnung (3, 30) für die Dialyselösung, welche in lösbarer Weise mit dem Dialysateingang des Generators verbunden ist, und wobei diese Einlaßöffnungen (2, 20) für das behandelte Wasser und die Auslaßöffnungen (3, 30) für die Dialyselösung auf gleichem Niveau des Behälters (1, 10) liegen, **dadurch gekennzeichnet, daß** letzterer Mittel zur Verteilung des Wassers im Volumen der festen Verbindung aufweist und seine Innenwand mindestens einen geneigten Bereich (8) aufweist, um eine Anhäufung der festen Verbindung zu verhindern.

2. Vorrichtung nach Anspruch 1, **dadurch gekennzeichnet, daß** die Mittel zur Verteilung des Wassers im Volumen der festen Verbindung durch einen Führungskanal (6, 60) gebildet werden.

3. Vorrichtung nach Anspruch 2, **dadurch gekennzeichnet, daß** der Führungskanal (6) im Inneren des Behälters (1) angeordnet ist.

4. Vorrichtung nach Anspruch 2, **dadurch gekennzeichnet, daß** der Führungskanal (60) außerhalb des Behälters (10) angeordnet ist.

5. Vorrichtung nach Anspruch 1, **dadurch gekennzeichnet, daß** der geneigte Bereich (8) einen Winkel zwischen 150° und 130° mit der Innenwand des Behälters bildet.

6. Vorrichtung nach Anspruch 2, **dadurch gekennzeichnet, daß** der Führungskanal (6) durch einen Bereich der Wand des Behälters, welche den oberen und den unteren Teil des letzteren verbindet, sowie durch eine innere Trennwand (7) von diesem gebildet wird, welche sich von den Rändern der Wassereintrittsöffnung (2) im wesentlichen parallel zu diesem Wandbereich erstreckt.

7. Vorrichtung nach Anspruch 1, **dadurch gekennzeichnet, daß** die Einlaßöffnungen (2, 20) für das behandelte Wasser and die Auslaßöffnungen (3, 30) für die Dialyselösung mit einem Filter versehen sind, dessen Porösität zwischen 1 und 100 µ liegt.

8. Vorrichtung nach einem der vorstehenden Ansprüche, **dadurch gekennzeichnet, daß** der Behälter eine Kapazität von 1 bis 10 Litern hat und 0,5 bis 6 kg einer festen Verbindung in Pulverform enthält.
